# EUROPÄISCHE PATENTANMELDUNG

(11) **EP 4 491 165 A1**
(43) Veröffentlichungstag der Anmeldung: **15.01.2025**
(21) Anmeldenummer: 24020230.9
(22) Anmeldetag: 09.07.2024
(51) Int. Cl.: A61F 13/0203, A61F 13/0206

(54) **WUNDAUFLAGE MIT EINER TAMPONADENEINHEIT UND VERWENDUNG EINER WUNDAUFLAGE**

(30) Priorität: 14.07.2023 DE 102023002882
(71) Anmelder: Heggemann, Timo, 50969 Köln (DE)
(72) Erfinder: Heggemann, Timo, 50969 Köln (DE)
(74) Vertreter: Köster, Christian

(57) **Zusammenfassung**

Dargestellt und beschrieben ist eine Wundauflage (1) mit einer Trägereinheit (2), einer auf einer ersten Seite der Trägereinheit (2) angeordneten Folieneinheit (3), einer zumindest abschnittsweise auf einer der ersten Seite gegenüberliegenden zweiten Seite der Trägereinheit (2) angeordneten Tamponadeneinheit (5), und einer auf der zweiten Seite der Trägereinheit (2) angeordneten Klebschicht (4), wobei sich die Tamponadeneinheit (5) von einem ersten Abschnitt (5.1), mit dem die Tamponadeneinheit an der Trägereinheit und/oder an der Folieneinheit befestigt ist, hin zu einem zweiten Abschnitt (5.2) erstreckt, der mit dem ersten Abschnitt (5.1) verbunden ist.

## Beschreibung

Die vorliegende Erfindung betrifft eine Wundauflage mit einer Tamponadeneinheit und eine Verwendung einer Wundauflage.

Aus dem Stand der Technik sind allgemein Wundauflagen und Tamponaden bekannt.

Einige von ihnen eigenen sich zur Behandlung von Abszessen oder Fisteln. Ein Abszess oder Fistel ist eine Ansammlung von Eiter in einem neu gebildeten Hohlraum im Gewebe. Häufig ist dieser Hohlraum unter der Haut. Aber auch an weiteren Körperstellen, wie dem Nabel oder der Peniswurzel, oder allgemein in Körperbereichen, die mit Haut oder Epithel bedeckt sind, können Abszesse oder Fisteln entstehen. Bei der Sinus pilonidalis tritt eine chronisch-entzündliche Erkrankung der Gesäßfalte auf. Durch die natürliche Behaarung in diesen Körperregionen, sowie die Lokalisation in Hautfalten, verheilen die Abszesse oder Fisteln in diesen Körperregionen in der Regel nur langsam, da in den Hautfalten ein feucht-warmes Milieu herrscht, in denen sich Bakterien und Pilze bevorzugt ansiedeln und vorteilhafte Bedingungen für eine Vermehrung vorfinden. Im Bereich der Gesäßfalte erfolgt zusätzlich häufig der Eintrag von Keimen aus dem Bereich des Afters. Dies macht die Behandlung von Abszessen und Fisteln oft aufwendig und langwierig. Nachdem ein Abszess oder eine Fistel geöffnet und fachgerecht gereinigt wurde sind die entstandenen Wundhöhlen vor dem Eintrag von weiteren Erregern, wie Bakterien, Pilzen, Viren, sowie anorganische Verunreinigungen zu schützen. Üblich ist daher das Einbringen einer Tamponade in die gereinigte Wundhöhle, die Wundexsudat aufnimmt und dann ein Verschluss der Wunde durch die Applikation einer flachen Wundauflage. Zusätzlich wird häufig ein Fixierverband angelegt, sofern dies überhaupt an der Körperstelle überhaupt möglich ist, um die Tamponade in die Wundhöhle zu pressen. Dementsprechend groß ist der Aufwand beim Wechseln der aus dem Stand der Technik bekannten Wundauflagen. Da der Wundheilungsprozess eines Abszesses oder einer Fistel ohnehin langwierig ist und regelmäßig mehrere Wochen beträgt bis sich die Wundhöhle von innen heraus verschlossen hat, ist die Wundversorgung durch Wundauflagen und ggf. Fixierverbände regelmäßig zu erneuern. Die regelmäßige Wundpflege ist auch wichtig, um Infektionen und Besiedelungen der Wunde durch Keime, Pilze und Vieren zu vermeiden. Dies würde den Wundheilungsprozess zusätzlich verzögern. Das Wechseln einer Wundauflage und das Herstellen eines Fixierverbandes erfordert in der Regel zwei Fachkräfte die arbeitsteilig die Wundhöhle spülen und reinigen, eine Tamponade in die Wundhöhle einbringen, eine neue Wundauflage aufbringen und einen Fixierverband anlegen.

Generell ist es bei der Behandlung von Wunden, wie sie bei Abszessen oder Fisteln entstehen, wünschenswert, diese einfach und zeiteffizient zu versorgen.

Daher ist die Aufgabe der vorliegenden Erfindung Wunden von Abszessen oder Fisteln einfach und zeiteffizient zu versorgen.

Gemäß einem ersten Aspekt der vorliegenden Erfindung wird die Aufgabe durch eine Wundauflage mit den Merkmalen des Patentanspruchs 1 gelöst. Die Wundauflage weist eine Trägereinheit, eine auf einer ersten Seite der Trägereinheit angeordneten Folieneinheit, eine zumindest abschnittsweise auf einer der ersten Seite gegenüberliegenden zweiten Seite der Trägereinheit angeordneten Tamponadeneinheit, und eine auf der zweiten Seite der Trägereinheit angeordneten Klebschicht auf. Die Tamponadeneinheit erstreckt sich von einem ersten Abschnitt, mit dem die Tamponadeneinheit an der Trägereinheit und/oder an der Folieneinheit befestigt ist, hin zu einem zweiten Abschnitt, der mit dem ersten Abschnitt verbunden ist.

Wie bereits beschrieben, weist die Wundauflage die Trägereinheit und die Folieneinheit und die Tamponadeneinheit und die Klebschicht auf. Das sind unterschiedliche Komponenten der Wundauflage die bevorzugt gezielt auf deren Funktion ausgebildet sein können.

Wie ebenfalls bereits beschrieben, weist die Wundauflage die Trägereinheit auf. An der Trägereinheit wird die Folieneinheit befestigt und die Klebschicht. Bevorzugt sind an der Trägereinheit die Folieneinheit und die Klebschicht befestigt. Alternativ kann an der Trägereinheit nur die Folieneinheit befestigt sein und zwischen Trägereinheit und Klebschicht befindet sich bevorzugt wenigstens eine weitere, mit der Trägerschicht und der Klebschicht in Kontakt stehende Schicht. Alternativ kann an der Trägereinheit nur die Klebschicht befestigt sein und zwischen Trägereinheit und der Folieneinheit befindet sich bevorzugt wenigstens eine weitere, mit der Trägerschicht und der Folieneinheit in Kontakt stehende Schicht. Die Trägereinheit ist die mechanisch tragende Komponente der hier beschriebenen Wundauflage. Die Trägereinheit besteht aus einem flächigen und flexiblen Material. Dadurch, dass die Trägereinheit flexibel ausgebildet ist, kann sich die Wundauflage an die Formgebung der zu behandelnden Körperstelle anpassen. Die Materialstärke der Trägereinheit beträgt zwischen 2 mm bis 6 mm. Eine dünnere Trägereinheit mit einer Materialstärke von 2 mm bis 4 mm kann den Tragekomfort für den Patienten verbessern, da ein dünneres Material weniger vom Körper absteht und weniger beim Tragen wahrgenommen wird. Darüber hinaus verringert eine dünne Trägereinheit die Wahrscheinlichkeit, dass ein Patient die Wundauflage teilweise vom Körper abstreift, beispielsweise indem Kleidung an einer Kante der Wundauflage hängen bleibt, wodurch diese teilweise von der Haut abgehoben wird. Ein weiterer Effekt einer dünnen Wundauflage ist, dass diese durch den Patienten besser unter Kleidung versteckt werden kann, wodurch die Lebensqualität des Patienten verbessert werden kann. Eine dickere Trägereinheit mit einer Materialstärke von 4 mm bis 6 mm hat den Vorteil, dass durch eine dickere Trägereinheit eine verbesserte Kompression des die Wunde umgebenden Gewebes erreicht wird, da die hier beschriebene Wundauflage überwiegend in Körperhöhlen bzw. -falten wie Achselhöhlen, Gesäßfalte oder Kniekehlen eingesetzt wird. In diesen Körperhöhlen wirkt während des Tragens durch einen Patienten eine Kraft auf die erste Seite der Trägereinheit und somit auf das die Wundhöhle umgebende Gewebe, wodurch überschüssige Wundexsudat aus dem umgebenden Gewebe sanft herausgedrückt wird und von der Tamponade der Tamponadeneinheit aufgenommen wird. So werden Entzündungen des Gewebes und Eiterbildung verringert oder ganz vermieden. Dies unterstützt den schnellen Verschluss der Wunde und somit allgemein die Wundheilung. Es sei angemerkt, dass dieser Vorteil auch bei einer dünnen Trägereinheit mit einer Dicke von 2 mm bis 4 mm auftritt, aber weniger stark ausgeprägt ist als bei einer dicken Trägereinheit.

Ein weiterer Vorteil einer dicken Trägereinheit besteht darin, dass die durch die Wundauflage zu schützende Wunde durch die Seitenkanten der Trägereinheit besser mit Sauerstoff versorgt werden kann und die Feuchtigkeit in der Wundhöhle sowie unter der Klebschicht besser reguliert wird, insbesondere kann die Feuchtigkeit, die unter der Klebschicht durch die Haut abgegeben wird, beispielsweise Schweiß, besser abgeführt werden. Dadurch wird erreicht, dass die Klebkraft der Klebschicht nicht durch Feuchtigkeitseintrag reduziert wird. Diese Vorteile treten insbesondere dann ein, wenn ein feuchtigkeitsregulierendes Material oder ein geschäumtes Material für die Trägereinheit verwendet wird.

Die Größe der Wundauflage ist so ausgebildet, dass sie die Wundhöhle mit einem Abstand von 1 cm bis 3 cm umrandet, wobei die Wundränder auf der Haut des Patienten von der Trägereinheit umschlossen werden. Dadurch wird sichergestellt, dass die hier beschriebene Wundauflage die Wunde vor äußeren Einflüssen und vor allem übermäßig vor Keimen, Erregern und anorganischen Verunreinigung schützt. Vorzugsweise ist die Wundauflage zwischen 4 cm bis 12 cm lang und zwischen 4 cm bis 12 cm breit. Sofern die Form der Wundauflage kreisförmig ist, beträgt der Radius der Wundauflage zwischen 2 cm bis 6 cm. Dadurch kann sichergestellt werden, dass die Wundöffnung zuverlässig umschlossen wird.

Wie ebenfalls bereits beschrieben, weist die Wundauflage die Folieneinheit auf. Die Folieneinheit ist auf der ersten Seite der Trägereinheit befestigt, mit anderen Worten auf der proximalen Seite der Trägereinheit. Die Folieneinheit deckt die Trägereinheit vorzugsweise vollständig, zumindest aber teilweise ab. Die Folieneinheit ist vorzugsweise eine Folie aus einem Polymer, z.B. Polypropylen oder Polyethylenterephthalat. Aber auch andere körperverträgliche Materialien können für die Folieneinheit eingesetzt werden. Die Folieneinheit hat vorzugsweise eine Dicke von 10 µm bis 150 µm. Die Folieneinheit ist vorzugsweise wasserundurchlässig ausgebildet. Besonders bevorzugt ist die Folieneinheit bakterien- und virenundurchlässig ausgebildet. Die Folieneinheit überdeckt auch die Ausnehmung, die von der Trägereinheit gebildet wird. Dadurch, dass die Folieneinheit die Ausnehmung überdeckt und auf der Oberseite, bzw. proximalen Seite, der Trägereinheit befestigt ist, wird die Wunde zuverlässig vor dem Eindringen von Bakterien, Viren und anderen Fremdstoffen von außen geschützt wodurch der Wundheilungsprozess unterstützt wird. Die Folieneinheit ist auf der Trägereinheit befestigt. Darüber hinaus erfolgt die Befestigung wenigstens in einem geschlossenen Umlauf, sodass eine umlaufende und in sich geschlossene Kontur gebildet wird, über die die Folieneinheit mit der Trägereinheit befestigt ist. Vorzugsweise ist die distale Seite der Folieneinheit auf der ersten Seite der Trägereinheit so mit der der Folieneinheit zugewandten Oberfläche der Trägereinheit auf der proximalen Seite der Trägereinheit verbunden, dass die der Folieneinheit zugewandte Oberfläche der Trägereinheit vollflächig mit zumindest einem Abschnitt der Folieneinheit verbunden ist und somit befestigt ist. Die Befestigung erfolgt vorzugsweise durch Heißsiegeln bzw. Aufschmelzen auf die Trägereinheit, sodass eine wasser- und luftdichte und dabei mechanisch zuverlässige Verbindung zwischen der proximalen Seite der Trägereinheit und der distalen Seite der Folieneinheit hergestellt wird. Eine Befestigung durch Heißsiegeln bzw. Aufschmelzen hat zudem die Vorteile, dass sich das Herstellungsverfahren für eine erfindungsgemäße Wundauflage vereinfacht, da keine zusätzlichen Klebstoffe benötigt werden und verringert somit auch den Material- und Ressourcenbedarf. Alternativ können auch wasserresistente hautverträgliche Kleber zum Befestigen der Folieneinheit auf der proximalen Seite der Trägereinheit eingesetzt werden. Auch der Einsatz von entsprechenden Primern, entweder auf der Folieneinheit oder Trägereinheit oder auf der Folien- und der Trägereinheit, kann vorteilhaft sein. Durch den Einsatz von Primern kann die Klebkraft erhöht werden und so sichergestellt werden, dass die Folieneinheit nach der Applikation auf dem Körper des Patienten beim Tragen luft- und wasserdicht mit der Trägereinheit verbunden bleibt. Eine Delamination kann also vermieden werden.

Besonders bevorzugt ist es, dass die Folieneinheit ein atmungsaktives Material ist. Die Folieneinheit bildet dabei eine Membrane. Dadurch kann ein gewisser Anteil an Wundexsudat durch die die Folieneinheit, die durch die Wundauflage abgedeckte Wundhöhle verlassen und durch die atmungsaktive Folieneinheit hindurch abdampfen bzw. verdunsten. Dadurch wird auch bei schwankender Menge oder viskösem Exsudat ein optimal feuchtes Wundheilungsmilieu erreicht.

Wie ebenfalls bereits beschrieben weist die Wundauflage die zumindest abschnittsweise auf der der ersten Seite gegenüberliegenden zweiten Seite der Trägereinheit angeordnete Tamponadeneinheit auf. Es ist bevorzugt, dass die Tamponadeneinheit aus einem saugfähigen und weichen Material oder Gewebe gebildet wird. Die Tamponadeneinheit besteht vorzugsweise aus Acetatfasern, Gaze oder anderen körperverträglichen und saugfähigen Materialen. Besonders bevorzugt kann es sein, dass die Tamponadeneinheit aus Alginaten besteht oder Alginate umfasst. Durch die Verwendung eines Alginats wird ein idealfeuchtes Wundmilieu erreicht. Dabei wird Wundexsudat durch die stark absorbierenden Eigenschaften des Alginats aufgenommen und Bakterien gebunden. Dadurch wird der Wundheilungsprozess unterstützt. Vorzugsweise ist das Alginat Calciumalginat. Bevorzugt sind die Tamponadeneinheit und die Trägereinheit zweiteilig ausgebildet. Bevorzugt sind die Tamponadeneinheit und die Trägereinheit bevorzugt abschnittsweise miteinander verbunden.

Ein Abschnitt der Tamponadeneinheit wird in die Wundhöhle eingebracht, um unter anderem ein Verschließen der Wundhöhle so lange zu verhindern, bis sie sich von distal nach proximal bis hin zur Körperoberfläche bzw. Hautseite verschlossen hat. Das bedeutet, dass Bestandteile der Tamponadeneinheit in die Wundhöhle eingeführt bzw. hineingestopft werden, wodurch sich dann Abschnitte der Tamponadeneinheit innerhalb der zu behandelnden Wundhöhle befinden. Wenn sich Abschnitte der Tamponadeneinheit in der Wundhöhle befinden, nimmt die Tamponadeneinheit Wundexsudat und Blut auf. Dies unterstützt den Heilungsprozess. Bevorzugt ist die Tamponadeneinheit keimarm und besonders bevorzugt steril ausgebildet.

Das Material der Tamponadeneinheit ist vorzugsweise nicht flusend. Darunter soll verstanden werden, dass sich keine Bestandteile der Tamponadeneinheit abtrennen oder ablösen, insbesondere, dass sich keine Fasern von der Tamponadeneinheit ablösen. Dadurch wird erreicht, dass keine Bestandteile der Tamponadeneinheit in der Wundhöhle verbleiben, wodurch Komplikationen bei der Wundheilung vermieden werden können. Dazu können bevorzugt gewebte Materialien aber auch Vliese eingesetzt werden, sofern sich keine Fasern aus dem Vlies lösen oder die Fasern des Vliesstoffes aneinander fixiert sind.

Die Tamponadeneinheit kann darüber hinaus mit einer antiseptischen Lösung getränkt oder beschichtet sein. Dadurch kann einer Wundinfektion vorgebeugt werden und das Wachstum von Keimen, Pilzen und Viren reduziert oder verhindert werden.

Besonders bevorzugt ist die Tamponadeneinheit mit einer bakterienbindenden Beschichtung aus Dialkylcarbamoylchlorid (DACC) beschichtet. Durch die bakterienbindende Eigenschaft der Beschichtung der Tamponadeneinheit wird erreicht, dass bei jedem Wechsel der hier beschriebenen Wundauflage am Patienten die Keimzahl in der Wundhöhle reduziert wird und der natürlichen Wundheilungsprozess bedeutend unterstützt wird. Besonders vorteilhaft ist dabei, dass auch multiresistente Keime wie z. B. MRSA oder VRE durch die mit Dialkylcarbamoylchlorid beschichtete Tamponadeneinheit gebunden werden. Dies ist insbesondere bei Patienten mit Fisteln oder einem vorliegenden Sinus pilonidalis (Steißbeinfistel) von Vorteil, da diese Patienten vermehrt vorgenannte Keime aufweisen und die Wundheilung häufig verlangsamt ist. Die vorgenannte bakterienbindende Beschichtung kann auch zusätzlich zu einer antiseptischen Beschichtung auf der Tamponadeneinheit aufgebracht sein. Dadurch werden die vorgenannten Vorteile einer antiseptischen Beschichtung und einer bakterienbindenden Beschichtung kombiniert.

Die Länge der Tamponadeneinheit beträgt vorzugsweise zwischen 5 cm bis 40 cm. Dadurch wird gewährleistet, dass ausreichend Tamponadeneinheit zum Auskleiden der Wundhöhle bereitgestellt wird. Insbesondere ist so sichergestellt, dass die Wundhöhle vollständig mit einem Abschnitt der Tamponadeneinheit ausgekleidet bzw. gefüllt werden kann. Um die Wundhöhle mit der Tamponadeneinheit zu füllen, wird ein Abschnitt der Tamponadeneinheit durch eine Fachkraft ziehharmonikaförmig oder leporelloartig in die Wundhöhle eingebracht. Dies kann beispielsweise mittels einer Pinzette erfolgen. Dies ist insbesondere dann erforderlich, wenn die in der Haut befindliche Wundöffnung nur einen kleinen Durchmesser aufweist, die darunter befindliche Wundhöhle jedoch bedeutend größer bzw. tief ausgebildet ist. Der in die Wundhöhle eingebrachte Abschnitt der Tamponadeneinheit kann aber auch einfach in die Wundhöhle hineingestopft werden.

Dadurch, dass die Länge der Tamponadeneinheit ist zwischen 5 cm bis 40 cm ist, ist ausreichend Material bereitgestellt, dass wenn die ein Abschnitt der Tamponadeneinheit in die Wundhöhle eingebracht ist, ein leichter Druck auf die Innenseite der Wundhöhle ausgeübt wird. Dabei kann die Breite des Materials der Tamponadeneinheit zwischen 0,5 cm und 5 cm betragen. Je breiter und länger das Material der Tamponadeneinheit ist, desto mehr Material steht zum Ausfüllen der Wundhöhle bereit. Dass die Tamponadeneinheit genügend Material bereitstellt, hat den Vorteil, dass zum einen sichergestellt ist, dass die Innenseiten der Wundhöhle in engem Kontakt mit dem eingebrachten Abschnitt der Tamponadeneinheit stehen und so zuverlässig Wundexsudat aufgenommen und abgeführt werden kann. Zum anderen wird durch den leichten Druck die Durchblutung des Wundgewebes gefördert, wodurch eine bessere Sauerstoff- und Nährstoffversorgung der Wunde erreicht wird. Für Wundhöhlen mit einer kleine Wundöffnung in der Haut oder dem Epithel eines Patienten, ist eine schmale Tamponadeneinheit, z. B. mit einer Breite von 0,5 cm - 2,5 cm, vorteilhaft. Eine schmalere Tamponadeneinheit hat den Vorteil, dass sie sich leichter durch eine kleine Wundöffnung in die Wundhöhle einbringen lässt. Für Wundhöhlen mit einer größeren Öffnung in der Haut oder dem Epithel eines Patienten oder einem großen Volumen der Wundhöhle, ist eine breitere Tamponadeneinheit, z. B. mit einer Breite ab 2,5 cm - 5 cm, vorteilhaft. Eine breitere Tamponadeneinheit hat den Vorteil, dass große Wundhöhlen effizient und schnell ausgefüllt werden können.

Wie ebenfalls bereits beschrieben weist die Wundauflage die auf der zweiten Seite der Trägereinheit angeordnete Klebschicht auf. Mittels der Klebschicht auf der zweiten Seite der Trägereinheit kann die Wundauflage auf der Haut des Patienten befestigt werden. Insbesondere dadurch, dass die Klebschicht auf der zweiten Seite der Trägereinheit angeordnet ist, wird gewährleistet, dass die Wundauflage auf der Haut des Patienten befestigt werden kann, ohne dass die Folieneinheit mit der Haut des Patienten in Kontakt kommen muss. Mit Hilfe der Klebschicht wird eine Befestigung der Wundauflage an der Haut eines Patienten bereitgestellt, wobei durch diese Befestigung die Wundhöhle feuchtigkeitsdicht verschlossen wird und vor dem Eindringen von Bakterien, Pilzen und Viren sowie anderen Fremdstoffen geschützt wird. Bevorzugt weist die Klebschicht einen silikonbasierten Klebstoff auf oder ist aus diesem gebildet.

Wie ebenfalls bereits beschrieben erstreckt sich die Tamponadeneinheit von dem ersten Abschnitt, mit dem die Tamponadeneinheit an der Trägereinheit und/oder an der Folieneinheit befestigt ist, hin zu dem zweiten Abschnitt, der mit dem ersten Abschnitt verbunden ist. Dadurch ist sichergestellt, dass die Tamponadeneinheit mindestens an einem Abschnitt fest an der Trägereinheit und/oder an der Folieneinheit befestigt ist, wodurch bei einem Wechsel der Wundauflage sichergestellt wird, dass die Tamponadeneinheit vollständig und rückstandslos aus der zu behandelnden Wundhöhle entfernt wird. Dadurch kann sichergestellt werden, dass nicht versehentlich einzelne Abschnitte einer mehrteiligen Tamponade in der Wundhöhle vergessen werden. Insbesondere ist es vorteilhaft, dass die Tamponadeneinheit Teil der Wundauflage ist, da es sich somit um ein einteiliges Gebilde handelt und nicht um separate Gebilde, nämlich eine klassische Tamponade, eine aus dem Stand der Technik bekannte Wundauflage und einen Fixierverband. Dadurch, dass die erfindungsgemäße Wundauflage eine Tamponadeneinheit aufweist, lässt sich die erfindungsgemäße Wundauflage besonders zeiteffizient und zuverlässig Anwenden und wechseln.

Vorteilhafterweise ist der erste Abschnitt der Tamponadeneinheit zwischen einem Abschnitt zwischen der Folieneinheit und der Trägereinheit befestigt. Die Befestigung kann mittels einer Klebeverbindung erfolgen, z. B. mittels eines Acrylatklebstoffes.

Alternativ kann der erste Abschnitt der Tamponadeneinheit auch mittels Ultraschallschweißen an der Trägereinheit befestigt sein. Dies hat den Vorteil, dass keine zusätzlichen Klebstoffe, die lösemittelhaltig sein können und zusätzlichen Zulassungsaufwand und Zusatzkosten für den Klebstoff selbst verursachen, eingesetzt werden müssen. Alternativ kann der erste Abschnitt der Tamponadeneinheit auch mittels Stanzverfahren oder Heißstanzverfahren zwischen der Trägereinheit und der Folieneinheit befestigt werden. Dadurch kann ebenfalls auf zusätzliche Kleber verzichtet werden mit den vorgenannten Vorteilen. In jedem Fall kann so eine ausreichend zugfeste Verbindung zwischen der Tamponadeneinheit und/oder der Trägereinheit und/oder der Folieneinheit hergestellt werden, um ein Ablösen der Tamponadeneinheit zu jeder Zeit zu verhindern.

Zusammenfassend kann also festgestellt werden, dass mit Hilfe der vorliegenden Erfindung Wunden von Abszessen und Fisteln einfach, zeiteffizient und zuverlässig versorgt werden können.

In einer Ausführungsform weist die Trägereinheit eine Ausnehmung auf, die durch einen Abschnitt der Trägereinheit definiert ist, der so um die Ausnehmung herum angeordnet ist, dass sich die Ausnehmung von der ersten Seite der Trägereinheit durch die Trägereinheit zu der zweiten Seite der Trägereinheit erstreckt. Durch die Ausbildung einer Ausnehmung in der Trägereinheit wird mit anderen Worten eine Trägereinheit mit einer Öffnung gebildet, die von dem Material der Trägereinheit gebildet wird. Die Formgebung der Ausnehmung ist nicht auf eine bestimmte Formgebung beschränkt. Vorzugsweise hat sie die gleiche oder eine ähnliche Kontur wie die Außenkontur der Trägereinheit selbst. Der Vorteil, der durch die Ausnehmung in der Trägereinheit erreicht wird, besteht darin, dass ein Hohlraum bereitgestellt wird, der überschüssiges Material der Tamponadeneinheit aufnehmen kann. Darüber hinaus ermöglicht die Ausnehmung eine visuelle Kontrolle der Tamponadeneinheit während der Anwendung an einem Patienten durch eine Fachkraft, sofern eine optisch transparente Folieneinheit eingesetzt wird. Ohne solch eine Ausnehmung würde die Wundauflage steif sein und eine optische Kontrolle der Tamponadeneinheit ohne einen zusätzlichen Indikator, z. B. Feuchtigkeitsindikator, nicht ermöglichen.

In einer Ausführungsform ist die Trägereinheit ringförmig, ellipsenförmig oder ein abgerundetes Rechteck. Wenn die Trägereinheit ringförmig ist, können insbesondere an flachen Körperregion ausgebildete Abszesse oder Fisteln, wie z. B. am Bauch oder Bauchnabel, behandelt werden, da die Wundhöhle sicher umschlossen wird. Wenn die Trägereinheit ellipsenförmig oder die Form eines abgerundeten Rechtecks aufweist, können Abszesse oder Fisteln insbesondere in Körperregion mit Körperfalten, wie z. B. in der Gesäßfalte oder einer Achselhöhle behandelt werden, da die Trägereinheit dann ausreichend Material bereitstellt, um in einer Körperfalte appliziert zu werden. Durch die Ellipsenform oder die Ausbildung der Trägereinheit als ein abgerundetes Rechteck können zudem die mechanischen Kräfte, die auf die Klebschicht zwischen der zweiten Seite der Trägereinheit und der Haut verringert werden. Dadurch wird das Risiko, dass sich die Klebeschicht partiell löst oder sich die Klebeschicht vollständig von der Haut eines Patienten ablöst verringert und sichergestellt, dass eine flüssigkeitsdichte Abdichtung zwischen Klebschicht und der Haut eines Patienten auch an Körperregionen mit Körperfalten bestehen bleibt.

In einer Ausführungsform weist die Trägereinheit ein geschäumtes Material auf oder ist aus einem geschäumten Material gebildet. Ein geschäumtes Material hat den Vorteil, dass es kompressibel ist und so Druckspitzen um die Wundöffnung herum verringert. Dabei wird auch eine Verbesserung der Rekapillarisierung des menschlichen Gewebes erreicht, was zu einer schnelleren und effizienteren Wundheilung führt. Weiterhin wird durch ein geschäumtes Material der Trägereinheit auch die Einbringung von Scherkräften auf die Klebschicht reduziert, da durch das Schaumgefüge der Trägereinheit Scherkräfte auf eine größere Fläche der Klebschicht verteilt werden und keine lokalen Scherkraftspitzen auftreten. Dies erhöht zum anderen dann auch den Tragekomfort für den Patienten, da die Wundauflage auch bei Bewegung des Patienten, sich diesen anpasst ein Reißen, Zerren und Ziehen an der Haut vermieden wird. Als Material eignen sich beispielsweise geschäumtes Polyurethan oder geschäumtes Acrylat. Wenn die Trägereinheit aus dem geschäumten Material gebildet ist, können die mechanischen Eigenschaften der Trägereinheit vollständig durch die Wahl des geschäumten Materials eingestellt werden, da in diesem Fall die Trägereinheit aus dem geschäumten Material besteht. Wenn die Trägereinheit das geschäumte Material aufweist, kann die Trägereinheit weitere Materialien aufweisen, die zusammen mit dem geschäumten Material die Trägereinheit bilden, wodurch die mechanischen Eigenschaften der Trägereinheit zusätzlich durch die weiteren Materialien eingestellt werden können.

In einer Ausführungsform ist das geschäumte Material offenzellig oder geschlossenzellig. Das geschäumte Material kann also offenzellig sein. Ein offenzellig geschäumtes Material hat den Vorteil, dass die Wunde auch von außen mit Sauerstoff in Kontakt gerät und so die Wundheilung verbessert unterstützt wird. Zudem kann Feuchtigkeit die Wundhöhle verlassen und so die Feuchtigkeitsregulierung der Wunde verbessern. Alternativ kann das das geschäumte Material auch geschlossenzellig sein. Ein geschlossenzellig geschäumtes Material hat den Vorteil, dass die Wunde vor dem Eindringen von Flüssigkeiten, Bakterien, Viren und Pilzen verhindert werden kann. Dadurch kann einer Infektion der Wunde durch äußere Einflüsse vermieden werden und die Wundheilung unterstützt werden.

In einer Ausführungsform ist die Folieneinheit optisch transparent ausgebildet. Im Zusammenhang mit der vorliegenden Erfindung ist unter optisch transparent eine optische Transparenz im sichtbaren Wellenlängenbereich zu verstehen. Eine optisch transparente Folieneinheit hat den Vorteil, dass Fachkräfte optisch und ohne die Wundauflage zu lösen, beurteilen können, ob die Tamponadeneinheit bereits mit Wundexsudat getränkt oder gar gesättigt ist und deren Aufnahmekapazität erschöpft ist, oder nicht. Dadurch kann ein unnötiges Lösen der Wundauflage vermieden werden, wodurch der Wundheilungsprozess nicht gestört wird. So kann beispielsweise bei einer regelmäßigen täglichen Kontrolle der Tamponadeneinheit durch die optisch transparente Folieneinheit hindurch, von einer Fachkraft entschieden werden, die Wundauflage einen weiteren Tag auf der Wunde zu belassen. Dies reduziert dann auch Abfall und Behandlungskosten.

Bevorzugt ist die Folieneinheit eine semipermeable Membran. Wenn die Folieneinheit eine semipermeable Membran ist, dann hat das den Vorteil, dass die Wundhöhle von proximal nach distal durch die Folieneinheit mit Sauerstoff versorgt wird, und dabei Bakterien, Pilze und Viren sowie andere Fremdstoffe und Flüssigkeiten die Folieneinheit nicht von proximal nach distal passieren können. Bevorzugt ist die Membran so ausgebildet ist, dass sie durchlässig für Sauerstoff ist, sodass die Wundhöhle mit Sauerstoff versorgt wird, was die Wundheilung fördert.

In einer Ausführungsform erstreckt sich die Folieneinheit derart, dass zumindest ein Abschnitt der Folieneinheit die Ausnehmung der Trägereinheit vollständig überdeckt. Dadurch wird gewährleistet, dass die Folieneinheit sicher und dichtend auf der Trägereinheit befestigt ist und die Wundhöhle vor dem Eindringen von Bakterien, Pilzen und Viren sowie anderen Fremdstoffen geschützt ist.

In einer Ausführungsform ist der zweite Abschnitt der Tamponadeneinheit an der Trägereinheit und/oder an der Folieneinheit befestigt. Dies hat den Vorteil, dass der erste und der zweite Abschnitt Tamponadeneinheit, bzw. die Enden der Tamponadeneinheit, an der Wundauflage befestigt sind. Dadurch wird vermieden das ein offenes bzw. loses Ende entsteht, von dem sich Bestandteile der Tamponadeneinheit lösen könnten und so in der Wundhöhle verbleiben könnten, z. B. Fasern an einem losen nicht befestigten Ende eines gewebten Tamponadenmaterials.

In einer Ausführungsform liegen der erste Abschnitt und zweite Abschnitt aneinander an. Dies hat den Vorteil, dass der erste und zweite Abschnitt der Tamponadeneinheit, bzw. die Enden der Tamponadeneinheit, eine Schlinge bilden und der erste und zweite Abschnitt der Tamponadeneinheit an der Wundauflage befestigt sind. Dadurch wird vermieden das ein offenes bzw. loses Ende entsteht, von dem sich Bestandteile der Tamponadeneinheit lösen könnten und so in der Wundhöhle verbleiben könnten. Dadurch muss die Wundhöhle weniger gründlich durch Fachpersonal auf Reste der Tamponadeneinheit kontrolliert werden, wodurch das Entfernen der erfindungsgemäßen Wundauflage effizient ist.

In einer Ausführungsform sind der erste Abschnitt und zweite Abschnitt voneinander beanstandet. Dies hat den Vorteil, dass der erste und zweite Abschnitt der Tamponadeneinheit, bzw. die Enden der Tamponadeneinheit, an der Wundauflage befestigt sind, sich der erste und zweite Abschnitt der Tamponadeneinheit aber nicht berühren. Dies hat herstellungstechnisch den Vorteil, dass der erste und zweite Abschnitt der Tamponadeneinheit beispielsweise nebeneinander aber beanstandet an der Trägereinheit und/oder der Folieneinheit befestigt werden. Dadurch wird dann zum einen vermieden, dass sich der erste und zweite Abschnitt der Tamponadeneinheit überlappen, wodurch Schwierigkeiten bei der Verklebung, bzw. dem Verschweißen der Folieneinheit auf der ersten Seite der Trägereinheit, also der proximalen Seite, vermieden werden können. Weiterhin hat dies den Vorteil, dass die Höhe reduziert wird, da die Tamponadeneinheit nicht als eine doppelte Lage, bzw. nahezu vollständig überlappend, zwischen der Folieneinheit und der Trägereinheit ausgeführt wird.

In einer Ausführungsform weist die Tamponadeneinheit einen rüschenförmigen Abschnitt auf. Unter rüschenförmig soll hier ein gekraustes, gefälteltes oder gekrepptes Gewebe verstanden werden. Dies hat den Vorteil, dass das Gewebe, welches den rüschenförmigen Abschnitt der Tamponadeneinheit bildet, besonders anschmiegsam an die Innenwand der Wundhöhle ausgebildet ist. Dadurch wird erreicht, dass der Abschnitt der Tamponadeneinheit, der in die Wundhöhle eingebracht wird, einen leichten Druck auf die Innenseite der Wundhöhle ausübt und sich dieser Druck besonders gleichmäßig auf die Innenwand verteilt. Dies hat den Vorteil, dass zum einen sichergestellt ist, dass die Innenseiten der Wundhöhle in engem Kontakt mit dem eingebrachten Abschnitt der Tamponadeneinheit stehen und so zuverlässig Wundexsudat aufgenommen und abgeführt werden kann. Zum anderen wird durch den leichten und gleichmäßigen Druck die Durchblutung des Wundgewebes gleichmäßig gefördert, wodurch eine bessere Sauerstoff- und/oder Nährstoffversorgung der Wunde erreicht wird.

Der rüschenförmige Abschnitt wird bereits beim Herstellungsprozess in das Tamponadenmaterial bzw. das Gewebe der Tamponadeneinheit eingebracht. Beispielsweise kann der rüschenförmige Abschnitt dadurch erzeugt werden, dass das Material in einer Form, z. B. mittels einer Wellenform, unter Wärmeeinwirkung geprägt oder plastisch verformt wird. Solch eine Formgebung lässt sich einfach und zuverlässig herstellen. Alternativ kann durch ein maschinelles Falten eines Teils der Tamponadeneinheit in einer Zick-Zack-Form ein rüschenförmiger Abschnitt erzeugt werden. Solch eine Zick-Zack-Form lässt sich besonders einfach maschinell herstellen. Sofern die Tamponadeneinheit aus einem gewebten Material besteht, beispielsweise einer Gaze, lässt sich ein rüschenförmiger Abschnitt auch durch die Variation der Maschenweite erreichen. Die so erzeugte rüschenform ist besonders anschmiegsam und weich, sodass die Wundinnenseite besonders gut und gleichmäßig kontaktiert werden kann.

Gemäß einem zweiten Aspekt der vorliegenden Erfindung wird die Aufgabe durch eine Verwendung mit den Merkmalen des Patentanspruchs 12 gelöst. Die Verwendung betrifft eine Verwendung einer Wundauflage gemäß dem ersten Aspekt zur Behandlung von Abszessen, Fisteln oder von Sinus pilonidalis.

Die erfindungsgemäße Wundauflage wird anhand von fünf Figuren schematisch dargestellt. Diese sind nicht maßstabsgetreu dargestellt. Gleiche Elemente sind mit gleiche Bezugszeichen versehen. Darüber hinaus sind die dargestellten Ausgestaltungen beispielhaft und sollen nicht die Allgemeinheit der Ansprüche beschränken.
- Figuren 1, 2 und 3: zeigen jeweils eine schematische Darstellung einer ersten Ausführungsform einer erfindungsgemäßen Wundauflage,
- Figuren 4 a) - f): zeigen schematische Darstellungen der erfindungsgemäßen Wundauflage,
- Figur 5: zeigt eine schematische Darstellung der Verwendung einer Ausführungsform einer erfindungsgemäßen Wundauflage.

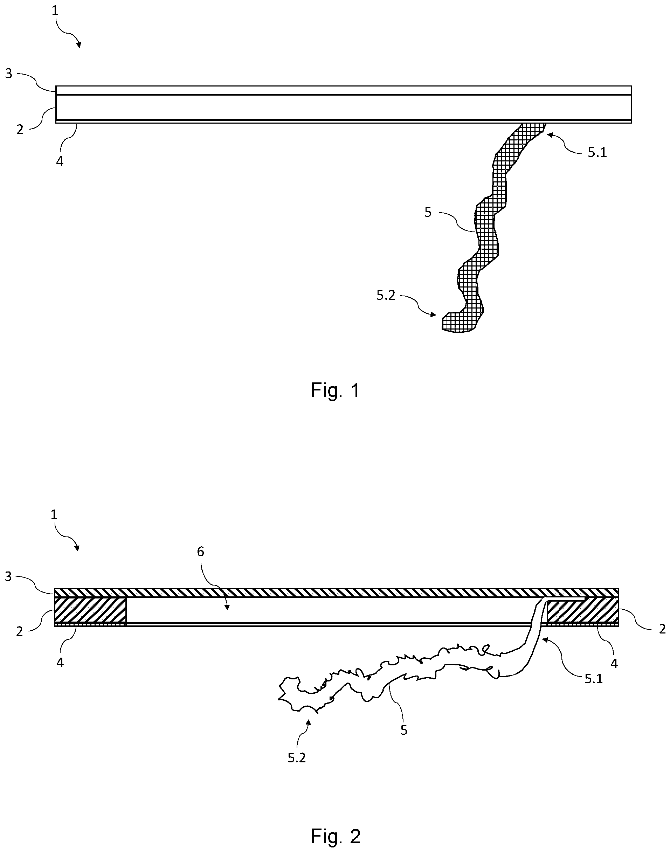

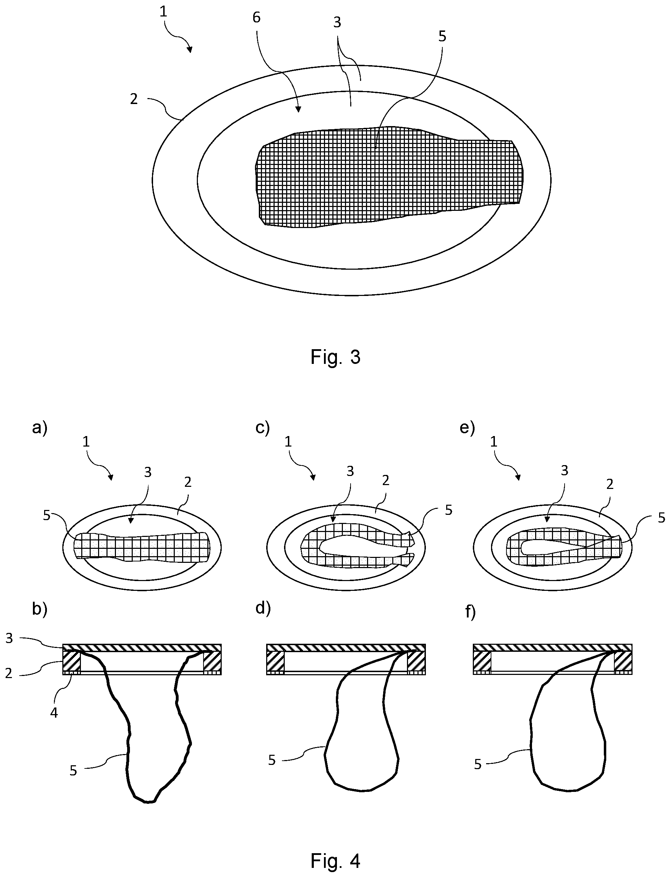

In den Figuren 1, 2 und 3 ist jeweils eine schematische Darstellung einer ersten Ausführungsform einer erfindungsgemäßen Wundauflage 1 dargestellt. Dabei zeigt Figur 1 eine Seitenansicht, Figur 2 eine Schnittdarstellung und die Figur 3 eine Draufsicht der ersten Ausführungsform der Wundauflage 1.

Die Wundauflage 1 weist eine Trägereinheit 2, eine Folieneinheit 3, eine Klebschicht 4 und eine Tamponadeneinheit 5 auf. An der Trägereinheit 2 sind die Folieneinheit 3 und die Klebschicht 4 befestigt. In einer alternativen Ausführungsform, die im Übrigen der ersten Ausführungsform der erfindungsgemäßen Wundauflage 1 entspricht, ist an der Trägereinheit 2 nur die Folieneinheit 3 befestigt. Dann befindet sich zwischen Trägereinheit 2 und Klebschicht 4 wenigstens eine weitere, mit der Trägereinheit 2 und der Klebschicht 4 in Kontakt stehende Schicht. In einer weiteren alternativen Ausführungsform, die im Übrigen der ersten Ausführungsform der erfindungsgemäßen Wundauflage 1 entspricht, ist an der Trägereinheit 2 nur die Klebschicht 4 befestigt. Dann befindet sich zwischen Trägereinheit 2 und der Folieneinheit 3 wenigstens eine weitere, mit der Trägerschicht 2 und der Folieneinheit 3 in Kontakt stehende Schicht.

Die Trägereinheit 2 weist eine Ausnehmung 6 auf, die durch einen Abschnitt der Trägereinheit 2 definiert ist, der so um die Ausnehmung 6 herum angeordnet ist, dass sich die Ausnehmung 6 von der ersten Seite der Trägereinheit 2 durch die Trägereinheit 2 zu der zweiten Seite der Trägereinheit 2 erstreckt. Durch die Ausbildung der Ausnehmung 6 in der Trägereinheit 2 wird mit anderen Worten eine Trägereinheit 2 mit einer Öffnung gebildet, die von dem Material der Trägereinheit 2 gebildet wird. Die Formgebung der Ausnehmung 6 ist nicht auf eine bestimmte Formgebung beschränkt. Vorzugsweise hat sie die gleiche oder eine ähnliche Kontur wie die Außenkontur der Trägereinheit selbst. Der Vorteil, der durch die Ausnehmung 6 in der Trägereinheit 2 erreicht wird, besteht darin, dass ein Hohlraum bereitgestellt wird, der überschüssiges Material der Tamponadeneinheit 5 aufnehmen kann. Darüber hinaus ermöglicht die Ausnehmung 6 eine visuelle Kontrolle der Tamponadeneinheit 5 während der Anwendung an einem Patienten durch eine Fachkraft, sofern eine optisch transparente Folieneinheit 5 eingesetzt wird. Ohne solch eine Ausnehmung 6 würde die Wundauflage 1 steif sein und eine optische Kontrolle der Tamponadeneinheit 5 ohne einen zusätzlichen Indikator, z. B. Feuchtigkeitsindikator, nicht ermöglichen.

Dadurch, dass die Trägereinheit 2 flexibel ausgebildet ist, kann sich die Wundauflage 1 an die Formgebung der zu behandelnden Körperstelle anpassen. Die Materialstärke der Trägereinheit 2 beträgt zwischen 2 mm bis 6 mm. Eine dünnere Trägereinheit 2 mit einer Materialstärke von 2 mm bis 4 mm kann den Tragekomfort für den Patienten verbessern, da ein dünneres Material weniger vom Körper absteht und weniger beim Tragen wahrgenommen wird. Darüber hinaus verringert eine dünne Trägereinheit 2 die Wahrscheinlichkeit, dass ein Patient die Wundauflage 1 teilweise vom Körper abstreift, beispielsweise indem Kleidung an einer Kante der Wundauflage 1 hängen bleibt, wodurch diese teilweise von der Haut abgehoben wird. Eine dickere Trägereinheit 2 mit einer Materialstärke von 4 mm bis 6 mm aufweist hat den Vorteil, dass durch eine dickere Trägereinheit 2 eine verbesserte Kompression des die Wunde umgebenden Gewebes erreicht wird, da die hier beschriebene Wundauflage 1 überwiegend in Körperhöhlen bzw. -falten wie Achselhöhlen, Gesäßfalte oder Kniekehlen eingesetzt wird. In diesen Körperhöhlen wirkt während des Tragens durch einen Patienten eine Kraft auf das umgebende Gewebe, wodurch überschüssige Wundexsudat aus dem umgebenden Gewebe sanft herausgedrückt wird und von der Tamponade der Tamponadeneinheit 5 aufgenommen werden kann. So werden Entzündungen des Gewebes und eine Eiterbildung verringert oder ganz vermieden. Dies unterstützt die Wundheilung. Es sei angemerkt, dass dieser Vorteil auch bei einer dünnen Trägereinheit 2 mit einer Dicke von 2 mm bis 4 mm auftritt, aber weniger stark ausgeprägt ist als bei einer dicken Trägereinheit 2.

Ein weiterer Vorteil einer dicken Trägereinheit 2 besteht darin, dass die durch die Wundauflage 1 zu schützende Wunde durch die Seitenkanten der Trägereinheit 2 besser mit Sauerstoff versorgt werden kann und die Feuchtigkeit in der Wundhöhle sowie unter der Klebschicht 4 besser reguliert wird, insbesondere kann die Feuchtigkeit, die unter der Klebschicht 4 durch die Haut abgegeben wird, beispielsweise Schweiß, besser abgeführt werden. Dadurch wird erreicht, dass die Klebkraft der Klebschicht 4 nicht durch Feuchtigkeitseintrag reduziert wird. Diese Vorteile treten insbesondere dann ein, wenn ein feuchtigkeitsregulierendes Material oder ein geschäumtes Material für die Trägereinheit 2 verwendet wird.

Dadurch wird sichergestellt, dass die hier beschriebene Wundauflage 1 die Wunde vor äußeren Einflüssen und vor allem vor Erregern, Keimen und anorganischen Verunreinigung schützt. Die Wundauflage 1 ist zwischen 4 cm bis 12 cm lang und zwischen 4 cm bis 12 cm breit. Sofern die Form der Wundauflage 1 kreisförmig ist, beträgt der Radius der Wundauflage 1 zwischen 2 cm bis 6 cm. Dadurch kann sichergestellt werden, dass die Wundöffnung zuverlässig umschlossen wird.

Die Trägereinheit 2 ist ellipsenförmig. In einer weiteren alternativen Ausführungsform ist die Trägereinheit 2 ein abgerundetes Rechteck. Wenn die Trägereinheit 2 ellipsenförmig ist, können Abszesse oder Fisteln insbesondere in Körperregion mit Körperfalten, wie z. B. in der Gesäßfalte oder einer Achselhöhle behandelt werden, da die Trägereinheit 2 dann ausreichend Material bereitstellt, um in einer Körperfalte appliziert zu werden. Durch die Ellipsenform können die mechanischen Kräfte, die auf die Klebschicht 4 zwischen der zweiten Seite der Trägereinheit 2 und der Haut verringert werden. Dadurch wird das Risiko, dass sich die Klebeschicht 4 partiell löst oder sich die Klebeschicht 4 vollständig von der Haut eines Patienten ablöst verringert und sichergestellt, dass eine flüssigkeitsdichte Abdichtung zwischen Klebschicht 4 und der Haut eines Patienten auch an Körperregionen mit Körperfalten bestehen bleibt. Die vorgenannten Vorteile, die eine ellipsenförmige Trägereinheit 2 treffen ebenso auf eine Trägereinheit 2 die die Form eines abgerundeten Rechtecks aufweist.

In einer alternativen Ausführungsform ist die Trägereinheit 2 ringförmig. Wenn die Trägereinheit 2 ringförmig ist, können insbesondere an flachen Körperregion ausgebildete Abszesse oder Fisteln, wie z. B. am Bauch oder Bauchnabel, behandelt werden, da die Wundhöhle 101 sicher umschlossen wird.

Die Trägereinheit 2 ist aus einem geschäumten Material gebildet. Wenn die Trägereinheit 2 aus dem geschäumten Material gebildet ist, können die mechanischen Eigenschaften der Trägereinheit 2 vollständig durch die Wahl des geschäumten Materials eingestellt werden, da in diesem Fall die Trägereinheit 2 aus dem geschäumten Material besteht. In einer alternativen Ausführungsform weist die Trägereinheit 2 ein geschäumtes Material auf. Wenn die Trägereinheit 2 das geschäumte Material aufweist, kann die Trägereinheit 2 weitere Materialien aufweisen, die zusammen mit dem geschäumten Material die Trägereinheit 2 bilden, wodurch die mechanischen Eigenschaften der Trägereinheit 2 zusätzlich durch die weiteren Materialien eingestellt werden können.

Ein geschäumtes Material hat den Vorteil, dass es kompressibel ist und so Druckspitzen um die Wundöffnung herum verringert. Zum einen wird durch ein geschäumtes Material der Trägereinheit 2 auch die Einbringung von Scherkräften auf die Klebschicht 4 reduziert, da durch das Schaumgefüge der Trägereinheit Scherkräfte auf eine größere Fläche der Klebschicht 4 verteilt werden und keine lokalen Scherkraftspitzen auftreten. Dies erhöht zum anderen dann auch den Tragekomfort für den Patienten, da die Wundauflage 1 auch bei Bewegung des Patienten, sich diesen anpasst ein Reißen, Zerren und Ziehen an der Haut vermieden wird.

Das geschäumte Material ist in der ersten Ausführungsform geschlossenzellig. Ein geschlossenzellig geschäumtes Material hat den Vorteil, dass die Wunde vor dem Eindringen von Flüssigkeiten, Bakterien, Viren und Pilzen verhindert werden kann. Dadurch kann einer Infektion der Wunde durch äußere Einflüsse vermieden werden und die Wundheilung unterstützt werden. In einer alternativen Ausführungsform ist das geschäumte Material offenzellig. Ein offenzellig geschäumtes Material hat den Vorteil, dass die Wunde auch von außen mit Sauerstoff in Kontakt gerät und so die Wundheilung verbessert wird. Zudem kann Feuchtigkeit die Wundhöhle 101 verlassen und so die Feuchtigkeitsregulierung der Wunde verbessern.

Die Folieneinheit 3 ist auf einer ersten Seite der Trägereinheit 2 angeordnet. Mit anderen Worten ist die Folieneinheit 3 auf der proximalen Seite der Trägereinheit 2 angeordnet. Die Folieneinheit 3 deckt die Trägereinheit 2 vollständig ab. Alternativ kann die Folieneinheit 3 die Trägereinheit 2 zumindest teilweise abdecken. Die Folieneinheit 3 ist vorzugsweise eine Folie aus einem Polymer, z. B. Polypropylen oder Polyethylenterephthalat. Aber auch andere körperverträgliche Materialien können für die Folieneinheit 3 eingesetzt werden. Die Folieneinheit 3 hat vorzugsweise eine Dicke von 10 µm bis 150 µm. Die Folieneinheit ist wasserundurchlässig. Darüber hinaus ist die Folieneinheit 3 auch bakterien- und virenundurchlässig. Die Folieneinheit 3 überdeckt auch die Ausnehmung 6, die von der Trägereinheit 2 gebildet wird. Dadurch, dass die Folieneinheit 3 die Ausnehmung 6 überdeckt und auf der Oberseite, bzw. proximalen Seite, der Trägereinheit 2 befestigt ist, wird die Wunde zuverlässig vor dem Eindringen von Bakterien, Viren und anderen Fremdstoffen von außen geschützt wodurch der Wundheilungsprozess unterstützt wird. Die Folieneinheit 3 ist auf der Trägereinheit 2 befestigt. Die Befestigung erfolgt vorzugsweise durch Heißsiegeln bzw. Aufschmelzen auf die Trägereinheit 2, sodass eine wasser- und luftdichte und dabei mechanisch zuverlässige Verbindung zwischen der proximalen Seite der Trägereinheit 2 und der distalen Seite der Folieneinheit 3 hergestellt wird. Eine Befestigung durch Heißsiegeln bzw. Aufschmelzen hat zudem die Vorteile, dass sich das Herstellungsverfahren für eine erfindungsgemäße Wundauflage 1 vereinfacht, da keine zusätzlichen Klebstoffe benötigt werden und verringert somit auch den Material- und Ressourcenbedarf. Alternativ können auch wasserresistente hautverträgliche Kleber zum Befestigen der Folieneinheit 3 auf der proximalen Seite der Trägereinheit 2 eingesetzt werden. Auch der Einsatz von entsprechenden Primern, entweder auf der Folieneinheit 3 oder Trägereinheit 2 oder auf der Folien- und der Trägereinheit, kann vorteilhaft sein. Durch den Einsatz von Primern kann die Klebkraft erhöht werden und so sichergestellt werden, dass die Folieneinheit 3 nach der Applikation auf dem Körper des Patienten beim Tragen luft- und wasserdicht mit der Trägereinheit 2 verbunden bleibt. Eine Delamination kann also vermieden werden.

In einer weiteren alternativen Ausführungsform ist die Folieneinheit 3 ein atmungsaktives Material. Die Folieneinheit 3 bildet also eine Membrane. Dadurch kann ein gewisser Anteil an Wundexsudat durch die Folieneinheit 3, die durch die Wundauflage 1 abgedeckte Wundhöhle 101 verlassen und durch die atmungsaktive Folieneinheit 3 hindurch abdampfen bzw. verdunsten. Dadurch wird auch bei schwankender Menge oder viskösem Exsudat ein optimal feuchtes Wundheilungsmilieu erreicht.

Die Folieneinheit 3 ist optisch transparent ausgebildet. Eine optisch transparente Folieneinheit 3 hat den Vorteil, dass Fachkräfte optisch und ohne die Wundauflage 1 zu lösen, beurteilen können, ob die Tamponadeneinheit 5 bereits mit Wundexsudat getränkt oder gar gesättigt ist und deren Aufnahmekapazität erschöpft ist, oder nicht. Dadurch kann ein unnötiges Lösen der Wundauflage 1 vermieden werden, wodurch der Wundheilungsprozess nicht gestört wird. So kann beispielsweise bei einer regelmäßigen täglichen Kontrolle der Tamponadeneinheit 5 durch die optisch transparente Folieneinheit 3 hindurch, von einer Fachkraft entschieden werden, die Wundauflage 1 einen weiteren Tag auf der Wunde zu belassen. Dies reduziert dann Abfall und Behandlungskosten.

Die Folieneinheit 3 erstreckt sich derart, dass zumindest ein Abschnitt der Folieneinheit 3 die Ausnehmung 6 vollständig überdeckt. Dadurch wird gewährleistet, dass die Folieneinheit 3 sicher und dichtend auf der Trägereinheit 2 befestigt ist und die Wundhöhle 101 vor dem Eindringen von Bakterien, Pilzen und Viren sowie anderen Fremdstoffen geschützt ist.

Die Folieneinheit 3 ist eine semipermeable Membran. Wenn die Folieneinheit 3 eine semipermeable Membran ist, dann hat das den Vorteil, dass die Wundhöhle 101 von proximal nach distal durch die Folieneinheit 3 mit Sauerstoff versorgt wird, und dabei Bakterien, Pilze und Viren sowie andere Fremdstoffe und Flüssigkeiten die Folieneinheit 3 nicht von proximal nach distal passieren können. Bevorzugt ist die Membran so ausgebildet ist, dass sie durchlässig für Sauerstoff ist, sodass die Wundhöhle 101 mit Sauerstoff versorgt wird, was die Wundheilung fördert.

Die Klebschicht 4 ist auf der zweiten Seite der Trägereinheit 2 angeordnet. Die Klebschicht 4 ist an der Trägereinheit 2 befestigt. Mittels der Klebschicht 4 auf der zweiten Seite der Trägereinheit 2 kann die Wundauflage 1 auf der Haut des Patienten befestigt werden. Die Klebschicht 4 ist vollflächig auf der zweiten Seite der Trägereinheit 2 aufgebracht. Die Klebschicht 4 bildet auf der zweiten Seite der Trägereinheit 2 wenigstens einen in sich geschlossenen Umlauf. Mit anderen Worten ist die Klebschicht 4 als ein Ring auf der zweiten Seite der Trägereinheit 2 ausgebildet und auf der zweiten Seite der Trägereinheit 2 aufgebracht. Insbesondere dadurch, dass die Klebschicht 4 auf der zweiten Seite der Trägereinheit 2 angeordnet ist, wird gewährleistet, dass die Wundauflage 1 auf der Haut des Patienten befestigt werden kann, ohne dass die Folieneinheit 3 mit der Haut des Patienten in Kontakt kommen muss. Mit Hilfe der Klebschicht 4 wird eine Befestigung der Wundauflage 1 an der Haut eines Patienten bereitgestellt, wobei durch diese Befestigung die Wundhöhle feuchtigkeitsdicht verschlossen wird und vor dem Eindringen von Bakterien, Pilzen und Viren sowie anderen Fremdstoffen geschützt wird. Bevorzugt weist die Klebschicht 4 einen silikonbasierten Klebstoff auf oder ist aus diesem gebildet.

Die Tamponadeneinheit 5 ist zumindest abschnittsweise auf einer der ersten Seite gegenüberliegenden zweiten Seite der Trägereinheit 2 angeordnet. Es ist bevorzugt, dass die Tamponadeneinheit 5 aus einem saugfähigen und weichen Material oder Gewebe gebildet wird. Die Tamponadeneinheit 5 besteht vorzugsweise aus Acetatfasern, Gaze oder anderen körperverträglichen und saugfähigen Materialen. Ein Abschnitt der Tamponadeneinheit 5 wird in die Wundhöhle 101 eingebracht, um unter anderem ein Verschließen der Wundhöhle 101 so lange zu verhindern, bis sie sich von distal nach proximal bis hin zur Körperoberfläche bzw. Hautseite verschlossen hat. Das bedeutet, dass Bestandteile der Tamponadeneinheit 5 in diese Wundhöhle 101 eingeführt bzw. hineingestopft werden, wodurch sich dann Abschnitte der Tamponadeneinheit 5 innerhalb der zu behandelnden Wundhöhle 101 befinden. Wenn sich Abschnitte der Tamponadeneinheit 5 in der Wundhöhle 101 befinden, nimmt die Tamponadeneinheit 5 Wundexsudat und Blut auf. Dies unterstützt den Heilungsprozess. Die Tamponadeneinheit 5 ist steril ausgebildet, in jedem Fall aber keimarm.

In einer weiteren Ausführungsform ist die Tamponadeneinheit 5 nicht flusend ausgebildet. Darunter soll verstanden werden, dass sich keine Bestandteile der Tamponadeneinheit 5 abtrennen oder ablösen. Dadurch wird erreicht, dass keine Bestandteile der Tamponadeneinheit 5 in der Wundhöhle 101 verbleiben, wodurch Komplikationen bei der Wundheilung vermieden werden können. Dazu wird ein gewebtes Material eingesetzt. Sofern sich keine Fasern aus einem Vlies lösen oder die Fasern des Vliesstoffes aneinander fixiert sind kann die Tamponadeneinheit 5 auch ein Vlies sein.

In einer weiteren Ausführungsform ist die Tamponadeneinheit 5 mit einer antiseptischen Lösung getränkt oder beschichtet. Dadurch kann einer Wundinfektion vorgebeugt werden und das Wachstum von Keimen, Pilzen und Viren reduziert oder verhindert werden.

In einer weiteren Ausführungsform ist die Tamponadeneinheit 5 mit einer bakterienbindenden Beschichtung aus Dialkylcarbamoylchlorid (DACC) beschichtet. Durch die bakterienbindende Eigenschaft der Beschichtung der Tamponadeneinheit 5 wird erreicht, dass bei jedem Wechsel der hier beschriebenen Wundauflage 1 am Patienten die Keimzahl in der Wundhöhle 101 reduziert wird und der natürlichen Wundheilungsprozess bedeutend unterstützt wird. Besonders vorteilhaft ist dabei, dass auch multiresistente Keime wie MRSA oder VRE durch die mit Dialkylcarbamoylchlorid beschichtete Tamponadeneinheit 5 gebunden werden. Dies ist insbesondere bei Patienten mit Abszessen oder einem vorliegenden Sinus pilonidalis (Steißbeinfistel) von Vorteil, da diese Patienten vermehrt vorgenannte Keime aufweisen und die Wundheilung häufig verlangsamt ist.

In einer weiteren Ausführungsform wird die vorgenannte bakterienbindende Beschichtung zusätzlich zu einer antiseptischen Beschichtung auf der Tamponadeneinheit 5 aufgebracht. Dadurch werden die vorgenannten Vorteile einer antiseptischen Beschichtung und einer bakterienbindenden Beschichtung kombiniert.

Die Länge der Tamponadeneinheit 5 beträgt zwischen 5 cm bis 40 cm. Dadurch wird gewährleistet, dass ausreichend Tamponadeneinheit 5 zum Auskleiden der Wundhöhle 101 bereitgestellt wird. Dabei ist die Breite des Materials der Tamponadeneinheit 5 zwischen 0,5 cm und 5 cm. Je breiter das Material der Tamponadeneinheit 5 ist, desto mehr Material steht zum Ausfüllen der Wundhöhle 101 bereit. Dass die Tamponadeneinheit 5 genügend Material bereitstellt, hat den Vorteil, dass zum einen sichergestellt ist, dass die Innenseiten der Wundhöhle 101 in engem Kontakt mit dem eingebrachten Abschnitt der Tamponadeneinheit 5 stehen und so zuverlässig Wundexsudat aufgenommen und abgeführt werden kann. Zum anderen wird durch den leichten Druck die Durchblutung des Wundgewebes gefördert, wodurch eine bessere Sauerstoff- und Nährstoffversorgung der Wunde erreicht wird. Für Wundhöhlen 101 mit einer kleine Wundöffnung in der Haut oder dem Epithel eines Patienten, ist eine schmale Tamponadeneinheit 5, z. B. mit einer Breite von 0,5 cm - 2,5 cm, vorteilhaft. Eine schmalere Tamponadeneinheit 5 hat den Vorteil, dass sie sich leichter durch eine kleine Wundöffnung in die Wundhöhle 101 einbringen lässt. Für eine Wundhöhle 101 mit einer größeren Öffnung in der Haut oder dem Epithel eines Patienten oder einem großen Volumen der Wundhöhle 101, ist eine breitere Tamponadeneinheit 5, z. B. mit einer Breite ab 2,5 cm - 5 cm, vorteilhaft. Eine breitere Tamponadeneinheit 5 hat den Vorteil, dass große Wundhöhlen 101 effizient und schnell ausgefüllt werden können.

Die Tamponadeneinheit 5 weist einen rüschenförmigen Abschnitt auf. Unter rüschenförmig soll hier ein gekraustes, gefälteltes oder gekrepptes Gewebe verstanden werden. Wenn die Tamponadeneinheit 5 einen rüschenförmigen Abschnitt aufweist, hat dies den Vorteil, dass das Gewebe, welches den rüschenförmigen Abschnitt der Tamponadeneinheit 5 bildet, besonders anschmiegsam an die Innenwand der Wundhöhle 101 ausgebildet ist. Dadurch wird erreicht, dass der Abschnitt der Tamponadeneinheit 5, der in die Wundhöhle 101 eingebracht wird, einen leichten Druck auf die Innenseite der Wundhöhle 101 ausübt und sich dieser Druck besonders gleichmäßig auf die Innenwand verteilt. Dies hat den Vorteil, dass zum einen sichergestellt ist, dass die Innenseiten der Wundhöhle 101 in engem Kontakt mit dem eingebrachten Abschnitt der Tamponadeneinheit 5 stehen und so zuverlässig Wundexsudat aufgenommen und abgeführt werden kann. Zum anderen wird durch den leichten und gleichmäßigen Druck, die Durchblutung des Wundgewebes gleichmäßig gefördert, wodurch eine bessere Sauerstoff- und/oder Nährstoffversorgung der Wunde erreicht wird.

Der rüschenförmige Abschnitt wird bereits beim Herstellungsprozess in das Material der Tamponadeneinheit 5 eingebracht. Beispielsweise kann der rüschenförmige Abschnitt dadurch erzeugt werden, dass das Material der Tamponadeneinheit 5 in einer Form, z. B. mittels einer Wellenform, unter Wärmeeinwirkung geprägt oder plastisch verformt wird. Solch eine Formgebung lässt sich einfach und zuverlässig herstellen. Alternativ kann ein rüschenförmiger Abschnitt in der Tamponadeneinheit 5 durch mehrfaches Falten eines Abschnitts der Tamponadeneinheit 5 in einer Zick-Zack-Form erzeugt werden. Solch eine Zick-Zack-Form lässt sich besonders einfach maschinell herstellen. Unter eine Zick-Zack-Form kann auch ein Leporello verstanden werden, also eine ziehharmonika-artige Faltung eines Abschnitts der Tamponadeneinheit 5.

Sofern die Tamponadeneinheit 5 aus einem gewebten Material besteht, beispielsweise einer Gaze, lässt sich ein rüschenförmiger Abschnitt auch durch die Variation der Maschenweite erreichen. Die so erzeugte rüschenform ist besonders anschmiegsam und weich, sodass die Wundinnenseite besonders gut und gleichmäßig kontaktiert wird.

Ein wesentlicher Vorteil der vorliegenden Erfindung ist, dass sich die Tamponadeneinheit 5 von einem ersten Abschnitt, mit dem die Tamponadeneinheit 5 an der Trägereinheit 2 und/oder an der Folieneinheit 3 befestigt ist, hin zu einem zweiten Abschnitt erstreckt, der mit dem ersten Abschnitt verbunden ist. Dadurch ist sichergestellt, dass die Tamponadeneinheit 5 mindestens an einem Abschnitt fest an der Trägereinheit 2 und/oder an der Folieneinheit 3 befestigt ist, wodurch bei einem Wechsel der Wundauflage 1 gewährleistet wird, dass die Tamponadeneinheit 5 vollständig und rückstandslos aus der zu behandelnden Wundhöhle 101 entfernt wird. Dadurch kann sichergestellt werden, dass nicht versehentlich einzelne Abschnitte einer mehrteiligen Tamponade in der Wundhöhle 101 vergessen werden. Insbesondere ist es vorteilhaft, dass die Tamponadeneinheit 5 Teil der Wundauflage 1 ist, da es sich somit um ein einteiliges Gebilde handelt und nicht um separate Gebilde, nämlich eine klassische Tamponade, eine aus dem Stand der Technik bekannte Wundauflage und einen Fixierverband. Dadurch, dass die erfindungsgemäße Wundauflage 1 eine Tamponadeneinheit 5 aufweist, lässt sich die erfindungsgemäße Wundauflage 1 besonders zeiteffizient und zuverlässig Anwenden und wechseln.

Der zweite Abschnitt 5.2 ist an der Trägereinheit 2 und an der Folieneinheit 3 befestigt. In einer alternativen Ausführungsform ist der zweite Abschnitt 5.2 an der Trägereinheit 2 befestigt und insbesondere nicht an der Folieneinheit 3. In einer weiteren alternativen Ausführungsform ist der zweite Abschnitt 5.2 an der Folieneinheit 3 befestigt und insbesondere nicht an der Trägereinheit 2. In jedem Fall trifft es zu, dass wenn der zweite Abschnitt 5.2 an der Trägereinheit 2 und/ oder an der Folieneinheit 3 befestigt ist, erreicht wird, dass ein offenes bzw. loses Ende der Tamponadeneinheit 5 entsteht, von dem sich Bestandteile der Tamponadeneinheit 5 lösen könnten und so in der Wundhöhle 101 verbleiben könnten, z. B. Fasern an einem losen nicht befestigten Ende eines gewebten Tamponadenmaterials. Dadurch muss die Wundhöhle 101 weniger gründlich durch Fachpersonal auf Reste der Tamponadeneinheit 5 kontrolliert werden, wodurch das Entfernen der erfindungsgemäßen Wundauflage 1 effizient ist.

Der erste Abschnitt 5.1 der Tamponadeneinheit 5 ist zwischen einem Abschnitt zwischen der Folieneinheit 3 und der Trägereinheit 2 befestigt. Die Befestigung kann mittels einer Klebeverbindung erfolgen, z. B. mittels eines Acrylatklebstoffes. Alternativ kann der erste Abschnitt 5.1 der Tamponadeneinheit 5 auch mittels Ultraschallschweißen an der Trägereinheit 2 befestigt sein. Dies hat den Vorteil, dass keine zusätzlichen Klebstoffe, die lösemittelhaltig sein können und zusätzlichen Zulassungsaufwand und Zusatzkosten für den Klebstoff selbst, aufgewendet werden müssen. Alternativ kann der erste Abschnitt 5.1 der Tamponadeneinheit 5 auch mittels Stanzverfahren oder Heißstanzverfahren zwischen der Trägereinheit 2 und der Folieneinheit 3 befestigt werden. Dadurch kann ebenfalls auf zusätzliche Kleber verzichtet werden mit den vorgenannten Vorteilen. In jedem Fall kann so eine ausreichend zugfeste Verbindung zwischen der Tamponadeneinheit 5 und/oder der Trägereinheit 2 und/oder der Folieneinheit 3 hergestellt werden, um ein Ablösen der Tamponadeneinheit 5 zu jeder Zeit zu verhindern.

Der erste Abschnitt 5.1 und der zweite Abschnitt 5.2 sind voneinander beanstandet. Dies hat den Vorteil, dass der erste und zweite Abschnitt 5.2 der Tamponadeneinheit 5, bzw. die Enden der Tamponadeneinheit 5, an der Wundauflage 1 befestigt sind, diese sich aber nicht berühren. Dies hat herstellungstechnisch den Vorteil, dass der erste und zweite Abschnitt 5.1, 5.2 der Tamponadeneinheit 5 beispielsweise nebeneinander aber beanstandet an der Trägereinheit 2 und/oder der Folieneinheit 3 befestigt werden. Dadurch wird dann zum einen vermieden, dass sich der erste und zweite Abschnitt 5.2, 5.2 der Tamponadeneinheit 5 überlappen, wodurch Schwierigkeiten bei der Verklebung, bzw. dem Verschweißen der Folieneinheit auf der ersten Seite der Trägereinheit 2 vermieden werden können. Weiterhin hat dies den Vorteil, dass die Höhe reduziert wird, da die Tamponadeneinheit 5 nicht als eine doppelte Lage, bzw. nahezu vollständig überlappend, zwischen der Folieneinheit 3 und der Trägereinheit 2 ausgeführt wird.

In einer alternativen Ausführungsform liegen der erste Abschnitt 5.1 und der zweite Abschnitt 5.2 aneinander an.

In den Figuren 4 a) - f) sind schematische Darstellungen der erfindungsgemäßen Wundauflage 1 dargestellt. Dabei zeigen Figuren 4 a) und 4 b) eine zweite Ausführungsform der Wundauflage 1, Figuren 4 c) und 4 d) eine dritte Ausführungsform der Wundauflage 1 und Figuren 4 e) und 4 f) eine vierte Ausführungsform der Wundauflage 1.

Figuren 4 a) und 4 b) zeigen eine zweite Ausführungsform der Wundauflage 1, wobei der erste Abschnitt 5.1 und zweite Abschnitt 5.2 der Tamponadeneinheit 5 voneinander beanstandet sind. Der erste Abschnitt 5.1 und zweite Abschnitt 5.2 sind im Wesentlichen gegenüberliegend an der Trägereinheit 2 und/oder an der Folieneinheit 3 befestigt. Der erste Abschnitt 5.1 und zweite Abschnitt 5.2 berühren sich dabei nicht und sind an unterschiedlichen Stellen befestigt. Dass der erste Abschnitt 5.1 und zweite Abschnitt 5.2 der Tamponadeneinheit 5 voneinander beanstandet sind hat den Vorteil, dass der erste und zweite Abschnitt 5.1, 5.2 der Tamponadeneinheit 5, bzw. die Enden der Tamponadeneinheit, an der Wundauflage 1 befestigt sind, sich der erste und zweite Abschnitt 5.1, 5.2 der Tamponadeneinheit 5 aber nicht berühren. Dies hat herstellungstechnisch den Vorteil, dass der erste und zweite Abschnitt 5.1, 5.2 der Tamponadeneinheit 5 beispielsweise nebeneinander aber beanstandet an der Trägereinheit 2 und/oder der Folieneinheit 3 befestigt werden. Dadurch wird dann zum einen vermieden, dass sich der erste und zweite Abschnitt 5.1, 5.2 der Tamponadeneinheit 5 überlappen, wodurch Schwierigkeiten bei der Verklebung, bzw. dem Verschweißen der Folieneinheit 3 auf der ersten Seite der Trägereinheit 2, also der proximalen Seite, vermieden werden können. Weiterhin hat dies den Vorteil, dass die Höhe reduziert wird, da die Tamponadeneinheit 5 nicht als eine doppelte Lage, bzw. nahezu vollständig überlappend, zwischen der Folieneinheit 3 und der Trägereinheit 2 ausgeführt wird.

Figuren 4 c) und 4 d) zeigen eine dritte Ausführungsform der Wundauflage 1, wobei der erste Abschnitt 5.1 und zweite Abschnitt 5.2 der Tamponadeneinheit 5 wie in der zweiten Ausführungsform voneinander beanstandet sind, der erste Abschnitt 5.1 und zweite Abschnitt 5.2 aber im Wesentlichen auf der gleichen Hälfte an der Trägereinheit 2 und/oder an der Folieneinheit 3 befestigt sind. Dass der erste Abschnitt 5.1 und zweite Abschnitt 5.2 der Tamponadeneinheit 5, wie in der zweiten Ausführungsform voneinander beanstandet sind hat den Vorteil, dass der erste und zweite Abschnitt der Tamponadeneinheit 5, bzw. die Enden der Tamponadeneinheit 5, an der Wundauflage 1 befestigt sind, sich der erste und zweite Abschnitt 5.1, 5.2 der Tamponadeneinheit 5 aber nicht berühren. Dies hat herstellungstechnisch den Vorteil, dass der erste und zweite Abschnitt 5.2 der Tamponadeneinheit 5 beispielsweise nebeneinander aber beanstandet an der Trägereinheit 2 und/oder der Folieneinheit 3 befestigt werden.

Dadurch wird dann zum einen vermieden, dass sich der erste und zweite Abschnitt 5.1, 5.2 der Tamponadeneinheit 5 überlappen, wodurch Schwierigkeiten bei der Verklebung, bzw. dem Verschweißen der Folieneinheit 3 auf der ersten Seite der Trägereinheit 2 vermieden werden können. Weiterhin hat dies den Vorteil, dass die Höhe reduziert wird, da die Tamponadeneinheit 5 nicht als eine doppelte Lage, bzw. nahezu vollständig überlappend, zwischen der Folieneinheit 3 und der Trägereinheit 3 ausgeführt wird.

Figuren 4 e) und f) zeigen eine vierte Ausführungsform der Wundauflage 1, wobei der erste Abschnitt 5.1 und zweite Abschnitt 5.2 der Tamponadeneinheit 5 aneinander anliegen. Dass der erste Abschnitt 5.1 und zweite Abschnitt 5.2 aneinander anliegen hat den Vorteil, dass der erste Abschnitt 5.1 und zweite Abschnitt 5.2 der Tamponadeneinheit 5, bzw. die Enden der Tamponadeneinheit 5, eine Schlinge bilden und der erste und zweite Abschnitt 5.1, 5.2 der Tamponadeneinheit 5 an der Wundauflage 1 befestigt sind. Dadurch wird vermieden das ein offenes bzw. loses Ende entsteht, von dem sich Bestandteile der Tamponadeneinheit 5 lösen könnten und so in der Wundhöhle 101 verbleiben könnten. Dadurch muss die Wundhöhle 101 weniger gründlich durch Fachpersonal auf Reste der Tamponadeneinheit 5 kontrolliert werden, wodurch das Entfernen der erfindungsgemäßen Wundauflage 1 effizient ist.

Für die zweite, dritte und vierte Ausführungsform gelten die vorgenannten Merkmale und Vorteile der ersten Ausführungsform analog.

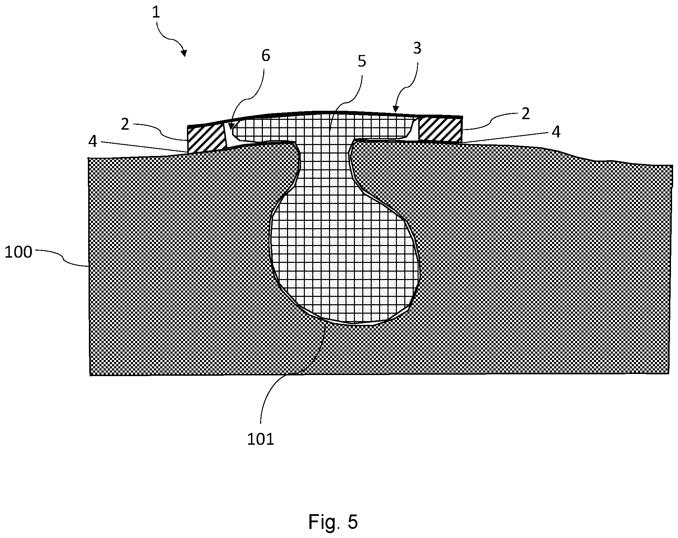

Figur 5 zeigt eine schematische Darstellung der Verwendung einer Ausführungsform einer erfindungsgemäßen Wundauflage 1 zur Behandlung von Abszessen, Fisteln oder von Sinus pilonidalis. Zur Behandlung von Abszessen, Fisteln oder von Sinus pilonidalis wird ein Abschnitt der Tamponadeneinheit 5 in die zuvor durch eine Fachkraft gereinigte Wundhöhle 101 die sich im Gewebe 100 des Patienten gebildet hat, hineingestopft. Die Wundhöhle 101 wird dabei vollständig mit einem Abschnitt der Tamponadeneinheit 5 ausgefüllt und übt leichten Druck auf die Innenwand der Wundhöhle 101 aus. Überschüssiges Material der Tamponadeneinheit 5 wird dann oberhalb der Wundhöhle 101 auf der Haut oder Epithel des Patienten platziert. Anschließend wird die Trägereinheit 2 und der daran befestigten Folieneinheit 3 mittels der Klebschicht 4 an auf der Haut oder dem Epithel des Patienten befestigt. Dabei wird die Trägereinheit 2 so ausgerichtet, dass die Ausnehmung 6 der Wundauflage 1, überschüssiges Material der Tamponadeneinheit 5 aufnimmt. Dabei übt der Abschnitt der Folieneinheit 3 der die Ausnehmung 6 überdeckt leichten Druck auf die in der Ausnehmung 6 befindliche Tamponadeneinheit 5 aus. Dadurch dass die Klebschicht 4 auf der Haut des Patienten aufgeklebt wird und überschüssiges Material der Tamponadeneinheit 5 vollständig von der Ausnehmung 6 aufgenommen wird, wird eine Abdichtung der Wundhöhle 101 gegenüber der äußeren Umgebung erreicht und ein Eindringen von Bakterien, Pilzen und Viren sowie anderen Fremdstoffen in die Wundhöhle 101 vermieden.

### Bezugszeichen

- 1: Wundauflage mit Tamponadeneinheit
- 2: Trägereinheit
- 3: Folieneinheit
- 4: Klebschicht
- 5: Tamponadeneinheit
- 5.1: Erster Abschnitt der Tamponadeneinheit
- 5.2: Zweiter Abschnitt der Tamponadeneinheit
- 6: Ausnehmung in Trägereinheit
- 100: Menschliches Gewebe
- 101: Wundhöhle

## Patentansprüche

1. Wundauflage (1) mit
einer Trägereinheit (2),
einer auf einer ersten Seite der Trägereinheit (2) angeordneten Folieneinheit (3), einer zumindest abschnittsweise auf einer der ersten Seite gegenüberliegenden zweiten Seite der Trägereinheit (2) angeordneten Tamponadeneinheit (5), und einer auf der zweiten Seite der Trägereinheit (2) angeordneten Klebschicht (4),
wobei sich die Tamponadeneinheit (5) von einem ersten Abschnitt (5.1), mit dem die Tamponadeneinheit an der Trägereinheit und/oder an der Folieneinheit befestigt ist, hin zu einem zweiten Abschnitt (5.2) erstreckt, der mit dem ersten Abschnitt (5.1) verbunden ist.

2. Wundauflage (1) nach dem vorhergehenden Anspruch, wobei die Trägereinheit (2) eine Ausnehmung (6) aufweist, die durch einen Abschnitt der Trägereinheit (2) definiert ist, der so um die Ausnehmung (6) herum angeordnet ist, dass sich die Ausnehmung (6) von der ersten Seite der Trägereinheit (2) durch die Trägereinheit (2) zu der zweiten Seite der Trägereinheit (2) erstreckt.

3. Wundauflage (1) nach einem der vorhergehenden Ansprüche, wobei die Trägereinheit (2) ringförmig, ellipsenförmig oder ein abgerundetes Rechteck ist.

4. Wundauflage (1) nach einem der vorhergehenden Ansprüche, wobei die Trägereinheit (2) ein geschäumtes Material aufweist oder aus einem geschäumten Material gebildet ist.

5. Wundauflage (1) nach einem der vorhergehenden Ansprüche, wobei das geschäumte Material offenzellig oder geschlossenzellig ist.

6. Wundauflage (1) nach einem der vorhergehenden Ansprüche, wobei die Folieneinheit (3) optisch transparent ausgebildet ist.

7. Wundauflage (1) nach einem der vorhergehenden Ansprüche, wobei sich die Folieneinheit (3) derart erstreckt, dass zumindest ein Abschnitt der Folieneinheit (3) die Ausnehmung (6) vollständig überdeckt.

8. Wundauflage (1) nach einem der vorhergehenden Ansprüche, wobei der zweite Abschnitt an der Trägereinheit (2) und/oder an der Folieneinheit (3) befestigt ist.

9. Wundauflage (1) nach einem der vorhergehenden Ansprüche, wobei der erste Abschnitt (5.1) und zweite Abschnitt (5.2) aneinander anliegen.

10. Wundauflage (1) nach einem der Ansprüche 1 bis 9, wobei der erste Abschnitt (5.1) und zweite Abschnitt (5.2) voneinander beanstandet sind.

11. Wundauflage (1) nach einem der vorhergehenden Ansprüche, wobei die Tamponadeneinheit (5) einen rüschenförmigen Abschnitt aufweist.

12. Verwendung einer Wundauflage (1) gemäß einem der vorhergehenden Ansprüche zur Behandlung von Abszessen, Fisteln oder von Sinus pilonidalis.
